(19) Europäisches Patentamt
European Patent Office
Office européen des brevets

(11) Veröffentlichungsnummer : **0 038 064**
**B1**

(12) # EUROPÄISCHE PATENTSCHRIFT

(45) Veröffentlichungstag der Patentschrift : **07.11.84**

(51) Int. Cl.³ : **G 01 N 31/10**, G 01 N 33/00, G 01 N 21/61

(21) Anmeldenummer : **81102823.2**

(22) Anmeldetag : **13.04.81**

(54) **Gasanalysator zur Bestimmung des Ammoniak-Anteils in einem Gasgemisch.**

(30) Priorität : **14.04.80 JP 48815/80**
**14.04.80 JP 50358/80 U**

(43) Veröffentlichungstag der Anmeldung : **21.10.81 Patentblatt 81/42**

(45) Bekanntmachung des Hinweises auf die Patenterteilung : **07.11.84 Patentblatt 84/45**

(84) Benannte Vertragsstaaten : **DE FR GB IT**

(56) Entgegenhaltungen :
**DE-A- 2 716 963**
**DE-A- 2 827 345**
**JP-A-55 031 953**
**JP-A-55 040 999**
**US-A- 3 977 836**
**Patents Abstracts of Japan Vol. 4, No. 47, 11. April 1980 Seite 150P6**

(73) Patentinhaber : **FUJI ELECTRIC CO. LTD.**
**1-1, Tanabeshinden, Kawasaki-ku**
**Kawasaki 210 (JP)**

(72) Erfinder : **Watanabe, Atsuo, Dipl.-Ing.**
**3-2-24-602 Asahigaoka**
**Hino-shi Tokyo (JP)**
Erfinder : **Kaneko, Teruo**
**2320 Kamikawa-cho**
**Hachioji-shi Tokyo (JP)**
Erfinder : **Tanaka, Takeo, Dipl.-Ing.**
**1-4-23 Naka**
**Kunitachi-shi Tokyo (JP)**
Erfinder : **Saito, Yoshio, Dipl.-Ing.**
**11-32-101 Motohashimoto-cho**
**Sagamihara-shi Kanagawa (JP)**

(74) Vertreter : **Mehl, Ernst, Dipl.-Ing.**
**Postfach 22 01 76**
**D-8000 München 22 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

**Beschreibung**

Die Erfindung bezieht sich auf einen Gasanalysator zur Bestimmung des Ammoniak-Anteils in einem Gasgemisch, insbesondere in Verbrennungsabgasen, mit einer Gasentnahme, einem daran angeschlossenen, gasleitenden Meßzweig mit einem $NH_3$/NO-Konverter, einen an die Gasentnahme angeschlossenen gasleitenden Vergleichszweig, eine aus beiden Zweigen beschickte NO-Meßeinrichtung zur Messung und Bestimmung des dem $NH_3$-Gehalt des Gasgemisches entsprechenden NO-Konzentrationsunterschiedes der aus dem Meßzweig und aus dem Vergleichszweig kommenden Gase. Ein derartiger Gasanalysator ist beispielsweise in der JP-A-55-40999 beschrieben.

Bei der Verbrennung von fossilen Brennstoffen enthält das Abgas neben Ammoniak große Anteile von Stickoxyden ($NO_x$) und/oder Schwefeltrioxyd ($SO_3$), welches mit Wasser reagiert und Schwefelsäurenebel bildet, die korrodierend auf Leitungen und Teile des Gasanalysators wirken.

Daneben können zwischen den einzelnen Gaskomponenten noch folgende sulfatbildende Reaktionen ablaufen :

$$SO_3 + NH_3 + H_2O \rightarrow (NH_4)HSO_4,$$
$$SO_3 + 2\ NH_3 + H_2O \rightarrow (NH_4)_2SO_4,$$
$$H_2SO_4 + NH_3 \rightarrow (NH_3)HSO_4,$$
und
$$H_2SO_4 + 2\ NH_3 \rightarrow (NH_4)_2SO_4$$

Die Sulfate kristallisieren üblicherweise bei einer Temperatur zwischen 200 und 250 °C.

Es besteht daher die Gefahr, daß sich in den gasführenden Leitungen und Teilen des Gasanalysators Sulfatkristalle niederschlagen, wodurch die Leitungswege verengt oder verstopft werden.

Eine naheliegende Lösung zur Umgehung dieser Schwierigkeiten besteht darin, die gasführenden Teile der Meßeinrichtung auf Temperaturen über 250 °C zu halten. Ein derartiges System ist jedoch kompliziert und aufwendig.

Es besteht demgemäß die Aufgabe, einen Ammoniak-Gasanalysator zu schaffen, bei dem die durch den Gehalt an $SO_3$ im Probengas auftretenden Schwierigkeiten mit geringem Aufwand vermieden werden.

Die Aufgabe wird gelöst durch einen Gasanalysator mit den im Anspruch 1 angegebenen Merkmalen.

In dem $NH_3$/NO-Konverter wird Ammoniak katalytisch in Stickoxyd und Wasserdampf umgewandelt :

$$NH_3 + 5/2\ O_2 \rightarrow NO + 3/2\ H_2O$$

Mit Hilfe der Schwefelsäurekonverter, die Kohlenstoff als Katalysator enthalten, werden Schwefelsäure, Sulfate und Schwefeltrioxyd in Schwefeldioxyd umgewandelt, gemäß folgenden Gleichungen :

$$H_2SO_4 + C \rightarrow SO_2 + CO_x,$$
$$(NH_4)HSO_4 + C \rightarrow SO_2 + NH_3 + CO_x + H_2O,$$
$$(NH_4)_2SO_4 + C \rightarrow SO_2 + 2\ NH_3 + CO_x + H_2O,$$
und
$$SO_3 + C \rightarrow SO_2 + CO_x,$$

wobei x = 1 oder 2 ist.

Die in den Schwefelsäurekonvertern gebildeten Gaskomponenten Schwefeldioxyd ($SO_2$) und Ammoniak ($NH_3$) kristallisieren bzw. fallen bei relativ niedrigen Temperaturen von etwa 50 °C in Form von Verbindungen aus.

Wird die Gastemperatur in und hinter den Schwefelsäurekonvertern im Meß- und Vergleichszweig darüber, etwa bei 70 °C, gehalten, wird eine Reaktion zwischen $SO_2$ und $NH_3$ vermieden.

Das ist im Vergleich mit der bereits genannten Thermostatisierung auf 200 bis 250 °C mit relativ einfachen Mitteln erreichbar, insbesondere durch doppelwandige oder anderweitig wärmeisolierte Rohrleitungen.

Weitere Merkmale und Vorteile sind in den abhäugigen Ansprüchen sowie in den Figuren und der dazugehörigen Beschreibung angegeben.

Figur 1 zeigt die vollständige Meßanordnung in schematischer Darstellung.

Figur 2 und Figur 3 zeigen ein Ausführungsbeispiel eines Schwefelsäurekonverters im Längs- und Querschnitt,

Figur 4 die in einem Anschlußstück vereinigten $NH_3$/NO- und Schwefelsäurekonverter.

Figur 1 In einen Gasraum 1, beispielsweise einem Schornstein, der das zu überwachende Gasgemisch, z. B. Verbrennungsabgase, führt, ragt eine Gasentnahme 10, die aus einem Filter 11, einem daran angeschlossenen Absaugrohr 12 und einem diese Teile umgebenden Schutzrohr 13 besteht, welche an einem Flansch 14 befestigt sind, der dazu dient, die Gasentnahme 10 in einer Öffnung in der Wand des Gasraums anzubringen.

Am Ausgang der Gasentnahme 10 sind parallel zwei gasleitende Zweige angeschlossen, nämlich ein Meßzweig 20 und ein Vergleichszweig 30.

Im Meßzweig 20 ist ein $NH_3$/NO-Konverter 21 angeordnet, der das in dem angesaugten Gasgemisch, kurz Probengas genannt, enthaltene Ammoniak in Stickstoffmonoxyd umwandelt.

Auf ihn folgt ein Schwefelsäurekonverter 50, in welchem die $SO_3$-haltigen Anteile im Probengas in Schwefeldioxyd ($SO_2$) umgewandelt werden. Falls das Probengas im Meßzweig 20 noch Stickstoffdioxyd ($NO_2$) enthält, kann ein $NO_2$/NO-Konverter 22 in den Meßzweig 20 eingefügt werden. Diesem folgt eine Pumpe 23, ein Gastrockner 24 und ein Filter 25.

In dem parallelen, ebenfalls probengasführenden Vergleichszweig 30 ist ein gleicher Schwefelsäurekonverter 60 angeordnet, gefolgt von einer Pumpe, einem Gastrockner 34 und einem Filter 35. Falls notwendig, kann auch im Vergleichszweig ein $NO_2$/NO-Konverter, ähnlich dem Konverter 22 im Meßzweig, eingefügt werden.

Beide Zweige 20 und 30 führen zu einer Meßeinrichtung 40 für Stickoxyd, die hier aus einem nichtdispersiven Infrarot-Gasanalysator besteht, dessen Aufbau und Betriebsweise im einzelnen als bekannt vorausgesetzt werden können. Er besteht im wesentlichen aus einem Infrarot-Strahler 41, einem durch den Motor 46 angetriebenen Strahlunterbrecher 47, einem Strahlenteiler 42, der die vom Strahler 41 ausgehende Strahlung in zwei gleichmäßige Strahlengänge, einen Meßstrahlengang und einen Vergleichsstrahlengang, aufteilt, einer im Meßstrahlengang liegenden Meßküvette 44 und einer parallel dazu im Vergleichsstrahlengang angeordneten Vergleichsküvette 43 sowie einem pneumatisch-elektrischen Detektor 45, dessen Empfängerkammern mit Stickoxyd (NO) gefüllt sind.

Das Probengas aus dem Meßzweig 20 wird durch die Meßküvette 44, das Probengas aus dem Vergleichszweig 30 durch die Vergleichsküvette 43 geführt.

Die durch den unterschiedlichen NO-Gehalt der Probengase aus dem Meß- und Vergleichszweig hervorgerufene unterschiedliche Strahlungsabsorption erzeugt in dem NO-sensibilisierten Detektor 45 ein entsprechendes Differenzsignal, welches dem Ammoniakgehalt des Gasgemisches im Gasraum 1 entspricht.

Eine bevorzugte Ausführungsform eines Schwefelsäurekonverters 50 oder 60 ist in Figur 2 im Längsschnitt, in Figur 3 im Querschnitt dargestellt.

Der Reaktionsraum 72 wird von einem dickwandigen Hohlzylinder 71 aus nicht korrodierendem Material, beispielsweise Titan, umschlossen. In ihm ist ein Katalysator 75, hier in Form einer von dem Probengas durchströmten Kohlegranulatschicht, angeordnet. In der Wand des Hohlzylinders 71 sind mehrere achsparallele, blind endende Bohrungen 73 in gleichen Winkelabständen angebracht. Sie dienen zur Aufnahme von hier nicht dargestellten elektrischen Heizelementen in Patronenform.

Eine weitere achsparallele und ebenfalls blind endende Bohrung 75 dient zur Aufnahme eines Temperaturfühlers, der über eine Meßleitung 86 an eine hier nicht gezeigte Temperaturregeleinrichtung angeschlossen ist, mit Hilfe derer die Temperatur im Reaktionsraum 72 auf etwa 400 °C gehalten wird.

Der Reaktionsraum 72 ist auf seiner Eingangsseite, links im Bild, mit einem auf die Stirnseite des Hohlzylinders 71 aufgeschraubten Flansch 76 ragt ein mit diesem verschweißtes Eingangsrohr 83 in den Reaktionsraum 72. Es ist von einem koaxial angeordneten Anschlußrohr 84 mit Abstand umgeben, welches mit einem Ende auf dem Flansch 76 festgeschweißt ist.

Die gegenüberliegende Ausgangsseite des Reaktionsraums 72 ist von einem mit der anderen Stirnseite des Hohlzylinders 71 verschraubten Flansch 78 abgeschlossen, dem eine Scheibe 77 aus hitzebeständigem Material, beispielsweise Asbest, unterlegt ist. In einer zentrischen Bohrung des Flansches 78 ist ein Ausgangsrohr 85 so befestigt, daß es ein kleines Stück in den Reaktionsraum 72 ragt. Der Flansch 78 ist darüber hinaus noch mit einer Reihe von Durchgangslöchern versehen zur Aufnahme und Durchführung der elektrischen Leitungen für die Heizelemente und den Temperaturfühler. Den Flanschen 76 und 78 sind beiderseits in Radialebenen Seitenwände 79 und 80 angefügt, an deren Rändern eine die Reaktionskammer umgebende zylindrische Hülse 82 befestigt ist. Der von der äußeren Mantelfläche des Hohlzylinders 71, der Hülse 82 und den Seitenwänden 79, 80 umgrenzte Hohlraum ist mit wärmedämmendem Material 81 gefüllt.

In Figur 4 ist eine konstruktive Ausführung einer an die Gasentnahme 10 angeschlossenen Leitungsverzweigung für Meßzweig 20 und Vergleichszweig 30 mit $NH_3$/NO-Konverter 21 und den beiden Schwefelsäurekonvertern 50 und 60 (siehe die Figuren 1, 2 und 3) dargestellt. Gleiche Teile sind mit gleichen Bezugszeichen versehen.

An die den Ausgang der Gasentnahme bildende Mittelbohrung in dem Flansch 14 ist koaxial ein Leitungsrohr 101, welches von einer zylindrischen Abdeckung 100 umgeben ist, angeschlossen. In dem Leitungsrohr 101 ist koaxial die von Wärmedämmungsmaterial 114 umgebene Reaktionskammer 102 des $NH_3$/NO-Konverters 21 angeordnet.

In der in ähnlicher Weise wie die Schwefelsäurekonverter gemäß Figur 2 und 3 ausgebildeten Reaktionskammer 102 in Form eines dickwandigen Hohlzylinders ist in dem Reaktionsraum 107 ein Platin-Katalysator 105 angeordnet. In der Wand der Reaktionskammer 102 sind gleichmäßig verteilt und achsparallel zum Reaktionsraum 107 Bohrungen 103 angeordnet, die zur Aufnahme von hier nicht dargestellten Heizelementen dienen, mit deren Hilfe der Platinkatalysator 105 auf Temperaturen zwischen 500 und 900 °C aufgeheizt wird.

Vor der Reaktionskammer 102 ist in der Wand des am Flansch 14 befestigten Leitungsrohrs 101 eine Öffnung 106 angebracht, in welche ein Anschlußrohr 95 eingeschweißt ist, welches den Anfang des Vergleichszweigs 30 bildet. Die Achse des Anschlußrohrs 95 und die Achse des Leitungsrohrs 101

schließen einen spitzen Winkel ein.

Das andere Ende des Anschlußrohrs 95 steckt in einer Gewindemuffe 96 mit einer ersten Überwurfmutter 97 und einer damit verspannbaren Dichtung 112. Von der anderen Seite her ist in die Gewindemuffe 96 das eingangsseitige Anschlußrohr 84 des Schwefelsäurekonverters 60 eingeschoben und in entsprechender Weise mit der zweiten Überwurfmutter 98 und der Dichtung 113 befestigt. Durch diese Rohrverbindung führt das Eingangsrohr 83 kleineren Durchmessers des Konverters 60 annähernd zum Ende des Leitungsrohrs 95 in der Öffnung 106. Mit Hilfe dieser doppelwandigen Rohranordnung wird eine Abkühlung des in den Vergleichszweig 20 eintretenden Probengases vermieden.

Aus dem $NH_3$/NO-Konverter 21 führt ein Anschlußrohr 90 zu dem eingangsseitigen Anschlußrohr 84 des Schwefelsäurekonverters 50 im Meßzweig 20 und ist mit diesem mittels der Gewindemuffe 91 und den von den Überwurfmuttern 92 und 93 angepreßten Dichtungen 110 und 111 verbunden. Die Länge des Eingangsrohrs 83 des Konverters 50 ist so bemessen, daß es in der Rohrverbindung 84, 90 bis vor den Ausgang der Reaktionskammer 102 des $NH_3$/NO-Konverters 21 führt. Es ist so eine sehr kompakte Bauweise mit kurzen Leitungswegen möglich, die eine Abkühlung des Probengases mit den geschilderten schädlichen Folgen verhindert.

**Ansprüche**

1. Gasanalysator zur Bestimmung des Ammoniak-Anteils in einem Gasgemisch, insbesondere in Verbrennungsabgasen, mit

— einer Gasentnahme (10),

— einem daran angeschlossenen, gasleitenden Meßzweig (20) mit

— einem $NH_3$/NO-Konverter (21),

— einem an die Gasentnahme (10) angeschlossenen gasleitenden Vergleichszweig (30),

— einer aus beiden Zweigen (20 und 30) beschickten NO-Meßeinrichtung (40) zur Messung und Bestimmung des dem $NH_3$-Gehalt des Gasgemisches entsprechenden NO-Konzentrationsunterschiedes der aus dem Meßzweig (20) und dem Vergleichszweig (30) kommenden Gase,

gekennzeichnet durch zwei Schwefelsäure-Konverter (50, 60) zur Umwandlung von Schwefelsäure, Schwefeltrioxyd und Ammonium-Sulfat in Schwefeldioxyd, von denen der eine (50) in dem Meßzweig (20) hinter dem $NH_3$/NO-Konverter (21), der andere in dem Vergleichszweig (30) nach der Gasentnahme (10) angeordnet ist.

2. Gasanalysator nach Anspruch 1, dessen Schwefelsäurekonverter (50, 60) gekennzeichnet sind durch :

a) einen Reaktionsraum (72) in einen dickwandigen Hohlzylinder (71) aus korrosionsfestem Material,

b) einen darin angeordneten Katalysator (75), vorzugsweise aus Kohlenstoff bestehend,

c) mehrere gleichmäßig um den Reaktionsraum (72) verteilte achsparallele und blind endende Bohrungen (73) in der Wand des Hohlzylinders (71), zur Aufnahme von elektrischen Heizelementen,

d) eine weitere achsparallele Bohrung (74) in der Wand des Hohlzylinders (71), zur Aufnahme eines Temperaturfühlers,

e) einen den Reaktionsraum (72) eingangsseitig abschließenden, an der einen Stirnseite des Hohlzylinders (71) befestigten Flansch (76) mit

f) einem in den Reaktionsraum (72) ragenden Eingangsrohr (83),

g) einem dieses koaxial umgebendes, auf dem Flansch (76) befestigtes Anschlußrohr (84),

h) einen den Reaktionsraum ausgangsseitig abschließenden, an der anderen Stirnseite des Hohlzylinders befestigten Flansch (78) mit einem aus dem Reaktionsraum führenden Ausgangsrohr (85) und Durchgangsbohrungen zur Durchführung elektrischer Anschlußleitungen für Heizelemente und Temperaturfühler.

3. Gasanalysator nach Anspruch 2, gekennzeichnet durch

a) an die Flansche (76 und 78) angesetzte, in Radialebenen verlaufende Seitenwände (79 und 80) größeren Durchmessers,

b) eine an deren Rändern befestigte, den Hohlzylinder (71) umgebende zylindrische Hülse (82),

c) währmedämmendes Material (81) in dem von der Hülse (82), den Seitenwänden (79, 80) und der äußeren Mantelfläche des Hohlzylinders (71) begrenzten Hohlraum.

4. Gasanalysator nach Anspruch 1 und 2, mit einem Leitungsrohr (101), welches an einem mit der Ausgangsöffnung der Gasentnahme (10) versehenen Flansch (14) befestigt ist und die koaxial angeordnete Reaktionskammer (102) des $NH_3$/NO-Konverters (21) enthält gekennzeichnet durch

a) ein von der Reaktionskammer (102) des $NH_3$/NO-Konverters (21) abgehendes Anschlußrohr (90),

b) Mittel (91, 92, 93, 110, 111) zur koaxialen Verbindung des Anschlußrohrs (90) mit dem eingangsseitigen Anschlußrohr (84) des Schwefelsäurekonverters (50) im Meßzweig (20), dessen Eingangsrohr (83) durch die Rohrverbindung (90, 84) bis zum Ausgang der Reaktionskammer (102) führt,

c) ein an eine vor der Reaktionskammer (102) liegende Öffnung (106) in der Wand des Leitungsrohrs (109) angeschlossenes Anschlußrohr (95), welches den Anfang des Vergleichszweigs (30) bildet, wobei die Mittelachsen von Leitungsrohr (101) und Anschlußrohr (95) einen spitzen Winkel einschließen,

d) Mittel (96, 97, 98, 112, 113) zur Verbindung des Anschlußrohrs (95) mit dem Anschlußrohr (84) des Schwefelsäurekonverters (60) im Vergleichszweig (30), dessen Eingangsrohr (83) durch die Rohrverbindung (84, 95) bis auf die Höhe der Öffnung (106) im Leitungsrohr (101) führt.

**Claims**

1. A gas analyser for determining the proportion of ammonia in a gas mixture, in particular in combustion gases, comprising
— a gas withdrawal point (10),
— a gas-conducting measuring branch (20) connected thereto and having,
— an $NH_3$/NO-converter (21),
— a gas-conducting comparison branch (30) connected to the gas withdrawal point (10),
— an NO-measuring device (40) which is fed from both branches (20 and 30) and serves to measure and determine the difference in NO-concentration of the gases coming from the measuring branch (20) and from the comparison branch (30), which difference corresponds to the $NH_3$-content of the gas mixture,
characterised by two sulphuric acid converters (50, 60) which serve to convert sulphuric acid, sulphuric trioxide and ammonium sulphate into sulphur dioxide, and one (50) of which is arranged in the measuring branch (20) after the $NH_3$/NO-converter (21), and the other one is arranged in the comparison branch (3) after the gas withdrawal point (10).

2. A gas analyser according to Claim 1, the sulphuric acid converters (50, 60) of which are characterised by :
a) a reaction space (72) in a thick-walled hollow cylinder (71) made of corrosion-resistant material,
b) a catalyst (75) arranged therein and which preferably consists of carbon,
c) a plurality of blind bores (73) parallel to the axis, which are evenly distributed around the reaction space (72) in the wall of the hollow cylinder (71), for the accommodation of electrical heating elements,
d) a further bore (74) parallel to the axis in the wall of the hollow cylinder (71) for the accommodation of a temperature sensor,
e) a flange (76) which closes the reaction space (72) at the input end and which is secured to the end face of the hollow cylinder (71), with
f) an inlet pipe (83) which projects into the reaction space (72),
g) a connecting pipe (84) which coaxially surrounds the inlet pipe and is secured to the flange (76),
h) a flange (78), which seals the reaction chamber at the output end and which is secured to the other end face of the hollow cylinder, with an outlet pipe (85) which leads out of the reaction space, and through-bores for the passage of electrical connection lines for heating elements and temperature sensors.

3. A gas analyser according to Claim 2, characterised by
a) side walls (79 and 80) extending in radial planes and of larger diameter, attached to the flanges (76 and 78),
b) a cylindrical sleeve (82) secured to the edges of said side walls and surrounding the hollow cylinder (71),
c) heat-insulating material (81) in the space bounded by the sleeve (82), the side walls (79, 80) and the outer surface of the hollow cylinder (71).

4. A gas analyser according to Claim 1 and 2, having a conduit pipe (101) which is secured to a flange (14) provided with the outlet opening of the gas withdrawal point (10) and which contains the coaxially-arranged reaction chamber (102) of the $NH_3$/NO-converter (21), characterised by
a) a connecting pipe (90) which branches off from the reaction chamber (102) of the $NH_3$/NO-converter (21),
b) means (91, 92, 93, 110, 111) for the coaxial connection of the connecting pipe (90) to the input-end connecting pipe (84) of the sulphuric acid converter (50) in the measuring branch (20), the input pipe (83) of which leads through the pipe connection (90, 84) to the output of the reaction chamber (102),
c) a connecting pipe (95), which is connected to an opening (106) in the wall of the conduit pipe (109), the opening being arranged before the reaction chamber (102), and which forms the start of the comparison branch (30), the central axes of the conduit pipe (101) and the connecting pipe (95) forming an acute angle,
d) means (96, 97, 98, 112, 113) for connecting the connecting pipe (95) to the connecting pipe (84) of the sulphuric acid converter (60) in the comparison branch (30), the input pipe (83) of which leads through the pipe connection (84, 95) to the level of the opening (106) in the conduit pipe (101).

**Revendications**

1. Analyseur de gaz pour déterminer la proportion d'ammoniac d'un mélange gazeux, notamment de gaz résiduaires de combustion, comprenant :

— un dispositif de prélèvement de gaz (10),

— une dérivation de mesure (20) qui s'y raccorde, dans laquelle passe le gaz et qui a

— un convertisseur $NH_3/NO$ (21),

— une dérivation de comparaison (30) dans laquelle passe du gaz et qui est raccordée au dispositif de prélèvement de gaz (10),

— un dispositif de mesure de NO (40) alimenté par les deux dérivations (20 et 30) et destiné à mesurer et à déterminer la différence de concentration de NO, correspondant à la teneur en $NH_3$ du mélange gazeux, entre les gaz sortant de la dérivation de mesure (20) et de la dérivation de comparaison (30), caractérisé en ce que par deux convertisseurs d'acide sulfurique (50, 60) pour transformer l'acide sulfurique, le trioxyde de soufre et le sulfate d'ammonium en dioxyde de soufre, dont l'un (50) est monté dans la dérivation de mesure (20) en aval du convertisseur $NH_3/NO$ (21), et dont l'autre est monté dans la dérivation de comparaison (30) après le dispositif de prélèvement de gaz (10).

2. Analyseur de gaz suivant la revendication 1, dont les convertisseurs d'acide sulfurique (50, 60) sont caractérisés par :

a) une chambre de réaction (72) dans un cylindre creux (71) à paroi épaisse en matériau résistant à la corrosion,

b) un catalyseur (75), de préférence en carbone, qui y est placé,

c) plusieurs perçages (73) borgnes, parallèles à l'axe et répartis d'une manière uniforme autour de la chambre de réaction, percés dans la paroi du cylindre creux (71) pour la réception d'éléments électriques de chauffage,

d) un autre perçage (74) parallèle à l'axe, percé dans la paroi du cylindre creux (71), pour la réception d'une sonde de température,

e) une bride (76) fixée à la partie frontale du cylindre creux (71), fermant la chambre de réaction (72) du côté de l'entrée et ayant

f) un conduit d'entrée (83) faisant saillie dans la chambre de réaction (72),

g) un conduit de raccordement (84) fixé à la bride (76) et entourant coaxialement le conduit d'entrée,

h) une bride (78) fixée à l'autre partie frontale du cylindre creux, fermant la chambre de réaction du côté de la sortie et ayant un conduit de sortie (85) menant hors de la chambre de réaction et des perçages traversant pour le passage de conducteurs électriques de raccordement des éléments de chauffage et de la sonde de température.

3. Analyseur de gaz suivant la revendication 2, caractérisé par

a) des parois latérales (79 et 80) de plus grand diamètre s'étendant dans des plans radiaux et mises sur les brides (76 et 78),

b) une douille (82) cylindrique entourant le cylindre creux (71) et fixée aux bords des parois latérales,

c) du matériau calorifuge (81) dans l'espace vide délimité par la douille (82), par les parois latérales (79 et 80) et par la surface latérale extérieure du cylindre creux (71).

4. Analyseur de gaz suivant la revendication 1 et 2, comprenant une conduite (101) qui est fixée à une bride (14) munie de l'ouverture de sortie du dsipositif de prélèvement de gaz et qui contient la chambre de réaction (102) disposée coaxialement du convertisseur $NH_3/NO$ (21), caractérisé par

a) un conduit de raccordement (90) partant de la chambre de réaction (102) du convertisseur $NH_3/NO$ (21),

b) des moyens (91, 92, 93, 110, 111) pour la liaison coaxiale du conduit de raccordement (90) avec le conduit de raccordement (84), se trouvant du côté de l'entrée du convertisseur d'acide sulfurique (50) de la dérivation de mesure (20), le tube d'entrée (83) du convertisseur menant par la liaison à conduits (90, 84) jusqu'à la sortie de la chambre de réaction (102),

c) un conduit de raccordement (95), qui est raccordé à une ouverture (106) se trouvant devant la chambre de réaction (102) et ménagée dans la paroi de la conduite (109), et qui forme le début de la dérivation de comparaison (30), les axes médians de la conduite (101) et du conduit de raccordement (95) faisant entre eux un angle aigu,

d) des moyens (96, 97, 98, 112, 113) pour mettre en communication le conduit de raccordement (95) avec le conduit de raccordement (84) du convertisseur d'acide sulfurique (60) dans la dérivation de comparaison (30), le conduit d'entrée (83) du convertisseur menant par la jonction à conduits (84, 95) jusqu'au niveau de l'ouverture (106) de la conduite (101).

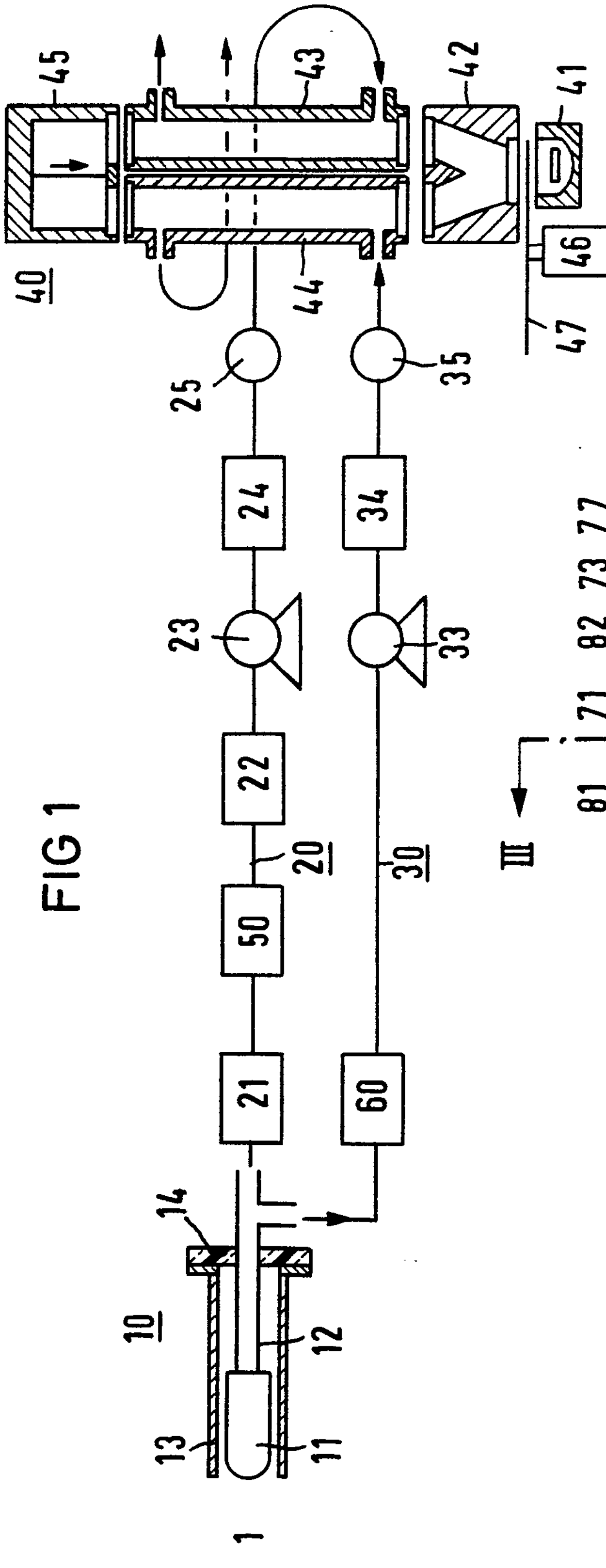


FIG 1

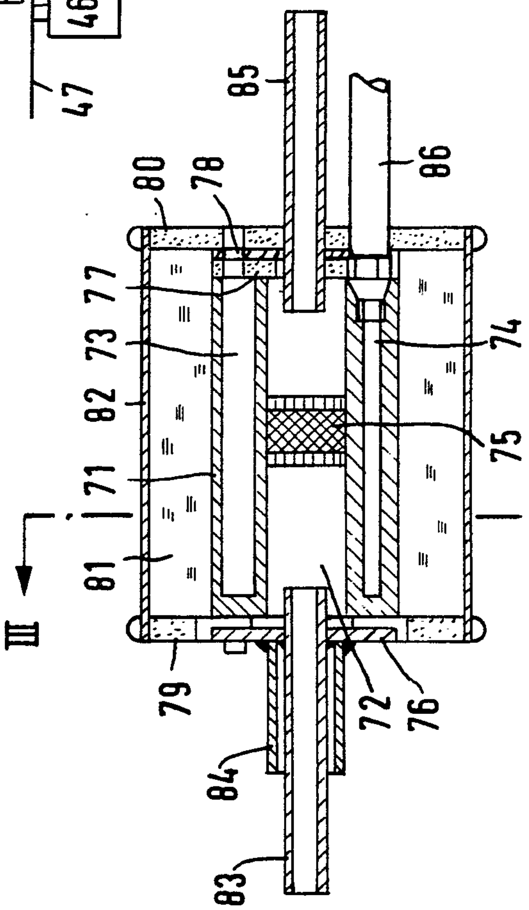


FIG 2

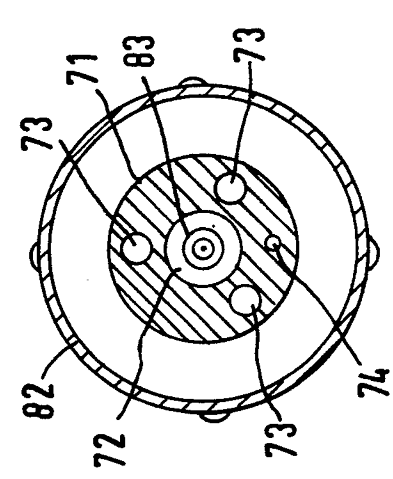


FIG 3

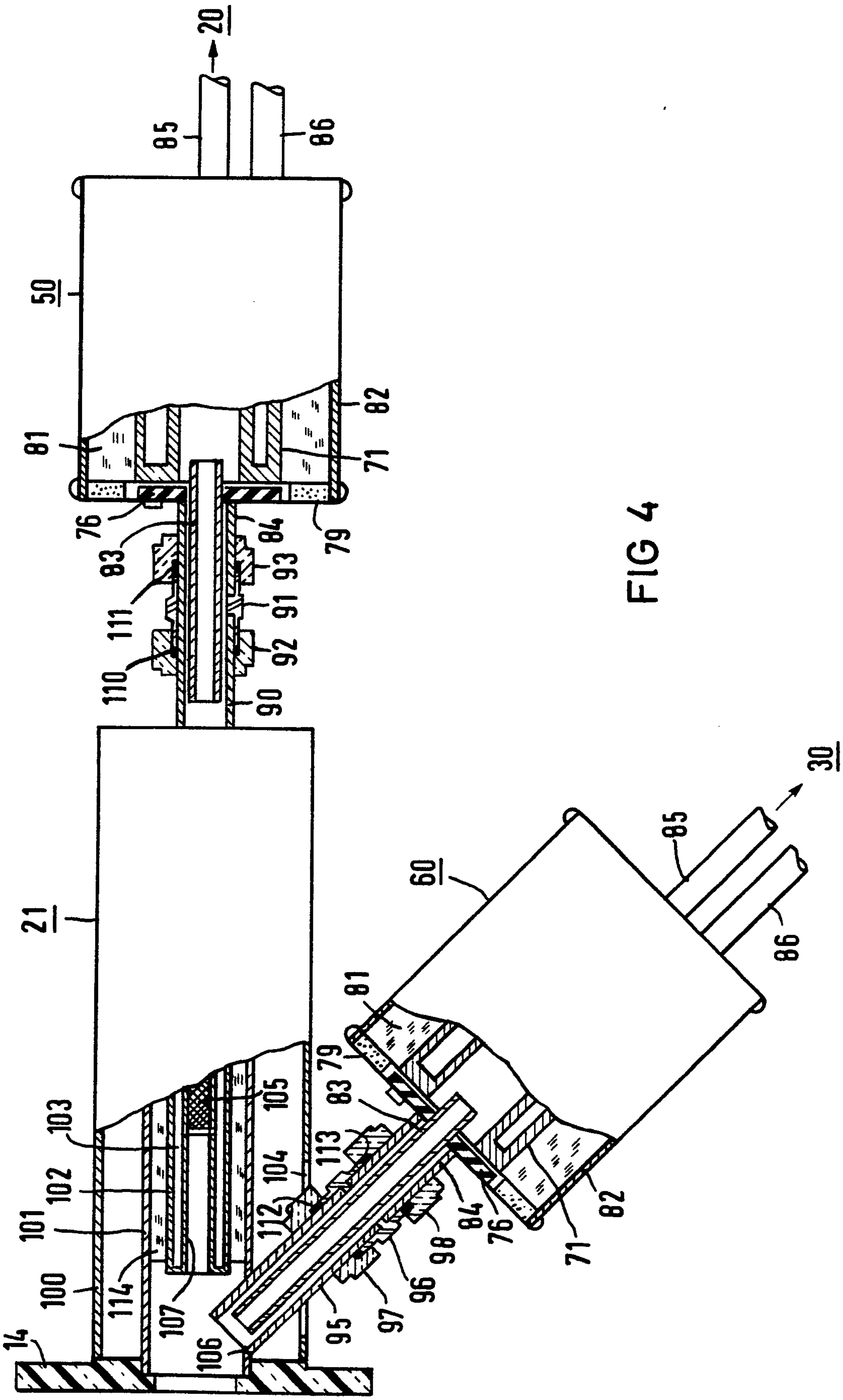

20
85
86
50
81
82
71
76
79
83
84
93
111
91
110
92
90
21
103
105
102
101
104
113
112
100
114
107
106
95
97
96
98
60
30
14


FIG 4